# EUROPEAN PATENT APPLICATION

(11) **EP 4 772 649 A1**
(43) Date of publication of application: **08.07.2026**
(21) Application number: 24859555.5
(22) Date of filing: 21.08.2024
(51) Int. Cl.: C12Q 1/02, C07K 14/705, C12N 5/071, C12N 5/10, C12N 15/12, G01N 33/15, G01N 33/50

(54) **SCREENING METHOD FOR ODOR-SUPPRESSING MATERIAL**

(30) Priority: 31.08.2023 JP 2023141659
(71) Applicant: Takasago International Corporation, Tokyo 144-8721 (JP)
(72) Inventor: MURAI Masato, Hiratsuka-shi, Kanagawa 254-0073 (JP); KASHIWAGI Takahiro, Hiratsuka-shi, Kanagawa 254-0073 (JP)
(74) Representative: Hoffmann Eitle
(86) International application number: PCT/JP2024/029557
(87) International publication number: WO 2025/047522

(57) **Abstract**

The present invention relates to a screening method for odor-suppressing materials. A screening method according to the present invention includes: causing a test substance and an odor-causing substance to act on at least one olfactory receptor polypeptide selected from the group consisting of olfactory receptor OR2AG2 and polypeptides that include an amino acid sequence having 80% or greater identity with the amino acid sequence of the aforementioned olfactory receptor and exhibit responsiveness to odor-causing substances; measuring the response of the olfactory receptor polypeptide to the odor-causing substance; and, on the basis of the measured response, identifying test substances that suppress a response of the olfactory receptor polypeptide as candidate substances for an odor-suppressing material.

## Description

### Technical Field

The present invention relates to a method for screening candidate substances for an odor-suppressing material.

### Background Art

In recent years, with the increased diversification of consumer preferences and living environments, the number of individuals who are sensitive to odors in their surroundings has been increasing. The range thereof extends from slight off-flavors present in foods to undesirable odors (malodors) in living spaces, and there is a need for the development of technologies capable of effectively reducing or deodorizing these odors (off-flavors and malodors) for the improvement of quality of life.

One of the most significant odors found in foods and living spaces is the "dust odor." A search for "taste dusty" on the Internet reveals that many people perceive this "dust odor" as an abnormal taste or off-flavor in various contexts. When this "dust odor" is present, it is perceived as dusty, earthy, grassy, powdery, medicinal, or moldy. For example, Patent Literature 1 describes the problem that in confectioneries such as chewing gum, in which the high-intensity sweetener aspartame is combined with certain fruit flavors, a "grassy flavor," which becomes dusty, earthy, powdery, medicinal, or moldy, i.e., a "dust odor," is generated over time. This "dust odor" is variously described by combinations of expressions such as earthy, dusty, musty/moldy, smoky, galbanum-like, bell pepper-like, vegetable-like, and green pealike. In the literature, it is often expressed in a two-word abbreviated form ("primary note-secondary note"), such as "earthy-dusty," "earthy-musty," or "dusty-moldy," and although understood at a sensory level, these expressions are not standardized due to their sensory nature.

As substances causing "dust odor," for example, pyridines or pyrazines generated through chemical reactions of amino acids, flavors, additives, and the like present in the food during the processing step are considered to be one of the main causative substances in processed foods. In addition to the processing steps, the "dust odor" may also be generated by the action of environmental microorganisms present in nature. For example, in drinking water, alkyl methoxypyrazines such as 2-isopropyl-3-methoxypyrazine and 2-isobutyl-3-methoxypyrazine, which are generated through metabolic degradation by environmental microorganisms present in ponds and rivers, may mix into the drinking water, resulting in the perception of a "dust odor" that is dusty, earthy, grassy, fishy, or moldy (Non Patent Literature 1). In addition, in living spaces, many publications have mentioned certain pyrazines contained in tobacco odor, grilled meat odor, cooking odor, kitchen odor, garbage odor, river odor, and the like as the cause of odors corresponding to "dust odor."

Although pyridines and pyrazines are considered to be the main causative substances of "dust odor," it has not been concluded that all "dust odors" are caused by pyridines or pyrazines, nor do all pyrazines necessarily have a "dust odor." According to Non Patent Literature 2, the odor of pyrazine compounds changes sensitively with slight differences in side chain structures, ranging from herb-like, burnt, nutty, chocolate-like, earthy, to grassy. In particular, pyrazines containing a methoxy group are known to exhibit a very grassy (bell pepper-like), dusty, and earthy odor, i.e., a "dust odor."

Since the occurrence of such "dust odor" impairs product value in foods and causes discomfort in living spaces, the search for flavoring materials that reduce the perceptions of "dust odor" is extremely important for improving the quality of life from the perspectives of food, clothing, and shelter. The method for searching for substances that reduce or deodorize such odors generally involves identifying the substances that cause the odor, and then having experts in odor evaluation individually assess a vast number of candidate substances for odor-suppressing materials by sensory evaluation. This common conventional method has the problem of low throughput. In recently established molecular biological techniques, olfactory receptors that respond to identified odor-causing substances are searched, and candidate substances for odor-suppressing materials that act as antagonists suppressing the response of those olfactory receptors are simultaneously screened *in vitro,* thereby enabling rapid identification of odor-suppressing materials by using the antagonists of olfactory receptors for specific odor-causing substances as odor blockers. However, in screening odor-suppressing agents that suppress specific olfactory receptor responses, it is generally not possible to identify the olfactory receptor that responds to the odor without identifying the substance causing the odor, and therefore identification of the odor-causing substance itself becomes the primary issue.

There are approximately 400 types of human olfactory receptors. Several olfactory receptors often respond to one odorant substance, and the odorant substance is distinguished through a combinatorial code involving the complex responses of narrowly-tuned olfactory receptors, which have high odor-note specificity and recognize specific odor notes and structures, and broadly-tuned olfactory receptors, which recognize a wide range of odorant substances without distinction (Non Patent Literature 3). Therefore, in the search for odor-suppressing materials, it is important to identify, among the several olfactory receptors that respond to an odor-causing substance, the olfactory receptor that is truly involved in recognizing the odor and is not a broadly-tuned olfactory receptor. For example, Non Patent Literature 4 reports that of the several olfactory receptors that respond to androstenone, olfactory receptor OR7D4 is the truly important olfactory receptor involved in the odor recognition of androstenone odor.

In addition, it has been reported that narrowly-tuned olfactory receptors often selectively respond to compounds that have somewhat similar structures, with varying degrees of response, and that they may exhibit odor-note specificity, where the same olfactory receptor responds to structurally different groups of compounds for a same odor note. For example, Non Patent Literature 5 shows that the narrowly-tuned olfactory receptor OR5AN1, which has extremely high odor-note specificity for musk scent, responds to both macrocyclic musks and nitro musks, which are groups of structurally different compounds, thereby demonstrating that compounds having the same primary note are recognized by the same olfactory receptor.

To date, no olfactory receptor has been identified as being clearly associated with "dust odor." However, Non Patent Literature 6 shows that the olfactory receptor OR2AG1 responds to 2-isopropyl-3-methoxypyrazine, 2-methoxy-3-(1-methylpropyl)pyrazine, and 2-isobutyl-3-methoxypyrazine, and that these substances have odor characteristics of so-called "dust odor," possessing grassy (green), musty, bell pepper-like, and galbanum-like odors. However, in Non Patent Literature 6, neither an *in vitro* receptor inhibition assay nor a sensory evaluation was conducted to determine whether "dust odor" is reduced by odorant substances (antagonists) that block the OR2AG1 response. Therefore, the possibility that "dust odor" is recognized by olfactory receptors other than OR2AG1 cannot be denied, and it remains uncertain whether OR2AG1 is linked to the odor note of "dust odor."

In addition, important odor substances having odor-note expressions similar to "dust odor," which are expressed as moldy, earthy, or muddy and are often expressed in two-word abbreviated forms such as "musty/moldy-earthy," include geosmin and 2-methylisoborneol. However, these odors are more accurately described as moldy based on their primary note and are of a different odor type from "dust odor." These two substances are known to specifically respond to the olfactory receptors OR11A1 and OR2M3, respectively (Patent Literature 2).

In addition, although pyrazines having side chain lengths of C1 to C2 are reported to respond to OR5K1 (Non Patent Literature 6, Patent Literature 3), only some of these pyrazines with C1 to C2 side chain lengths that trigger OR5K1 response possess odor characteristics of "dust odor."

### Citation List

### Patent Literature

Patent Literature 1: Japanese Patent Laid-Open No. 2009-131228
Patent Literature 2: Japanese Patent Laid-Open No. 2014-235098
Patent Literature 3: Japanese Translation of PCT International Application Publication No. 2015-512032

### Non Patent Literature

Non Patent Literature 1: Journal of Chromatography A, 1428 (2016), pp. 72-85
Non Patent Literature 2: Shigeharu Inoue, Microbes and Fragrance: The Power of Aroma in the Microscopic World, pp. 60-61 (2002)
Non Patent Literature 3: Journal of Neuroscience, 22 June, 2011, 31 (25), pp. 9179-9191
Non Patent Literature 4: Nature, 2007 Sep 27; 449(7161): pp. 468-72
Non Patent Literature 5: The Journal of Neuroscience, April 20, 2016, 36(16): pp. 4482-44
Non Patent Literature 6: ECRO XXV Istanbul: "Functional characterization of human olfactory receptors responding to pyrazine odorants". September 1, 2015, Posters
Non Patent Literature 7: Nature Communications 10:209 (2019)
Non Patent Literature 8: J Vis Exp. 2019 Apr 23; (146): 10.3791/59446

### Summary of Invention

### Technical Problem

Although "dust odor" is an important odor present in foods and living environments, only some of its causative substances are known, and its chemical entity remains unclear. In addition, although olfactory receptors that respond to some of the compounds having odor characteristics of "dust odor" have been disclosed, it remains unclear whether these receptors are narrowly-tuned olfactory receptors that are truly associated with the recognition of "dust odor."

Under such circumstances, it is desired to elucidate the chemical entity of "dust odor," identify olfactory receptors that specifically respond to "dust odor," and provide a method for efficiently screening candidate substances for a "dust odor"-suppressing material and the like.

### Solution to Problem

The present invention has been made in consideration of the above circumstances, and provides a method for screening candidate substances for an odor-suppressing material as described below.
[1] A method for screening odor-suppressing materials, the method comprising:
   causing a test substance and an odor-causing substance to act on at least one olfactory receptor polypeptide selected from the group consisting of olfactory receptor OR2AG2 and polypeptides that comprise an amino acid sequence having 80% or more identity with the amino acid sequence of the olfactory receptor and that are responsive to odor-causing substances;
   measuring the response of the olfactory receptor polypeptide to the odor-causing substance; and
   on the basis of the measured response, identifying a test substance that suppresses the response of the olfactory receptor polypeptide as a candidate substance for an odor-suppressing material.
[2] The method according to [1], wherein the odor is an odor expressed as "dust odor,"
   has at least one odor characteristic selected from the group consisting of dusty, and optionally, earthy, grassy, powdery, medicinal, and moldy,
   is an odor that comprises, as a primary note, at least one expression selected from the group consisting of "dusty," "earthy," and "grassy (bell pepper-like or vegetable-like)," or expressions similar thereto, as a descriptor expressing the odor characteristic, and
   optionally, is an odor that causes discomfort to humans.
[3] The method according to [2], wherein the odor is an odor that comprises, as a primary note, at least one expression selected from the group consisting of "dusty," "grassy (bell pepper-like)" and "grassy (vegetable-like)" as a descriptor expressing the odor characteristic.
[4] The method according to [3], wherein the odor is an odor that comprises, as a primary note, at least one expression selected from the group consisting of "dusty" and "grassy (bell pepper-like)" as a descriptor expressing the odor characteristic.
[5] The method according to any one of [1] to [4], wherein the odor is an odor that triggers olfactory receptor OR2AG2 response.
[6] The method according to any one of [1] to [5], wherein the odor-causing substance comprises one or more selected from the group consisting of compounds represented by the following formula (1) or (2): 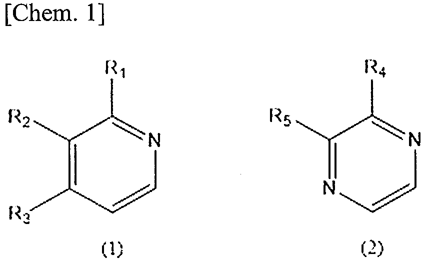
   wherein in formula (1), R₁ represents a hydrogen atom, an unbranched alkyl group having 1 to 3 carbon atoms, an optionally branched alkyl group having 4 to 6 carbon atoms, or an alkoxy group having 1 to 3 carbon atoms; R₂ and R₃ each independently represent a hydrogen atom, an optionally branched alkyl group having 1 to 6 carbon atoms, or an alkoxy group having 1 to 3 carbon atoms; and
   in formula (2), R₄ and R₅ each independently represent a hydrogen atom, an optionally branched alkyl group having 1 to 6 carbon atoms, or an alkoxy group having 1 to 3 carbon atoms.
[7] The method according to any one of [1] to [6], wherein the response of the olfactory receptor polypeptide to the odor-causing substance is measured on cells that endogenously express the olfactory receptor, or on cells genetically engineered to artificially express the olfactory receptor.
[8] The method according to any one of [1] to [7], wherein the response of the olfactory receptor polypeptide is measured by a reporter assay or calcium imaging.

### Advantageous Effects of Invention

By using the method of the present invention, it is possible to screen candidate substances for a "dust-odor"-suppressing material that can inhibit the binding of substances that cause the "dust odor," an important odor in foods and living spaces, to the olfactory receptor OR2AG2.

According to one embodiment of the present invention, the chemical entity of an odor that is sensorially, abstractly, or subjectively expressed as "dust odor" has been identified as an odor that triggers olfactory receptor OR2AG2 response. Therefore, even when the "dust odor" is caused by an unknown substance, by utilizing the fact that the causative substance that is invariably present in a mixture exhibiting "dust odor" binds to the olfactory receptor OR2AG2, it is possible to screen candidate substances for a "dust-odor"-suppressing material without identifying the substance that causes the "dust odor."

### Brief Description of Drawings

[Figure 1] Figure 1 is a diagram showing the results of olfactory receptor screening for 2-isobutyl-3-methoxypyrazine.
[Figure 2] Figure 2 is a diagram based on the screening of the responses of olfactory receptor OR2AG2 to 1,367 odorant substances, which shows the proportion of those that responded with a) a Fold Increase value of 3 or more for OR2AG2, and b) a Fold Increase value of 50 or more for OR2AG2, and the primary note proportion thereof.
[Figure 3] Figure 3 is a diagram showing the results of measuring the concentration-dependent responses of olfactory receptor OR2AG2 to a) 3-ethyl-2-pentylpyridine (3E2P-pyridine), b) 2-methoxy-3-isopropylpyrazine (2M3IP-pyrazine), c) 2-isobutyl-3-methoxypyrazine (2IB3M-pyrazine), and d) 2-sec-butyl-3-methoxypyrazine (2sB3M-pyrazine).
[Figure 4] Figure 4 is a diagram showing the results of measuring the concentration-dependent inhibition of OR2AG2 response by various antagonists of olfactory receptor OR2AG2 for a) 3-ethyl-2-pentylpyridine, b) 2-methoxy-3-isopropylpyrazine, c) 2-isobutyl-3-methoxypyrazine, and d) 2-sec-butyl-3-methoxypyrazine.
[Figure 5] Figure 5 is a diagram showing sensory test results indicating that various antagonists of olfactory receptor OR2AG2 suppress "dust odor" for a) 3-ethyl-2-pentylpyridine (3E2P-pyridine), b) 2-methoxy-3-isopropylpyrazine (2M3IP-pyrazine), c) 2-isobutyl-3-methoxypyrazine (2IB3M-pyrazine), and d) 2-sec-butyl-3-methoxypyrazine (2sB3M-pyrazine).
[Figure 6] Figure 6 is a diagram showing the results of measuring the concentration-dependent responses of olfactory receptor OR2AG2 to an extract exhibiting a "dust odor" of unknown components.
[Figure 7] Figure 7 is a diagram showing the results of measuring the concentration-dependent inhibition of OR2AG2 response by antagonists of olfactory receptor OR2AG2 for an extract exhibiting a "dust odor" with unknown components.
[Figure 8] Figure 8 is a diagram showing sensory test results indicating that an antagonist of olfactory receptor OR2AG2 suppresses the "dust odor" for an extract exhibiting a "dust odor" with unknown components.

### Description of Embodiments

### Summary of Present Invention

In the present invention, by identifying and utilizing olfactory receptor OR2AG2, which exhibits a specific response to the substances causing an odor expressed as a "dust odor," a method for efficiently screening candidate substances for a "dust-odor"-suppressing material, and thereby a "dust-odor"-suppressing composition that reduces the unpleasantness of the "dust odor," are provided.

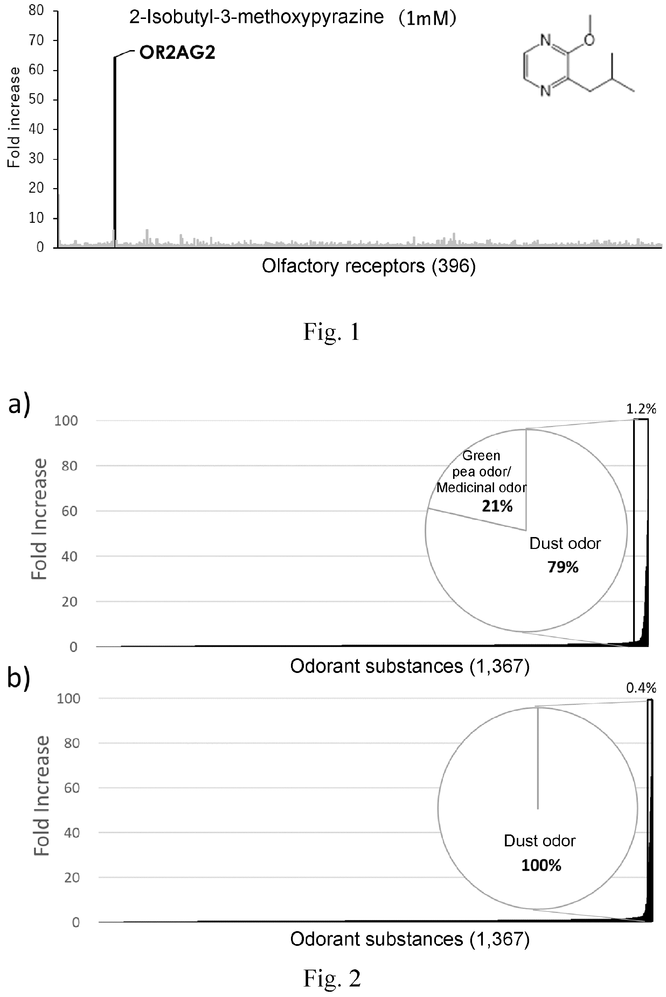

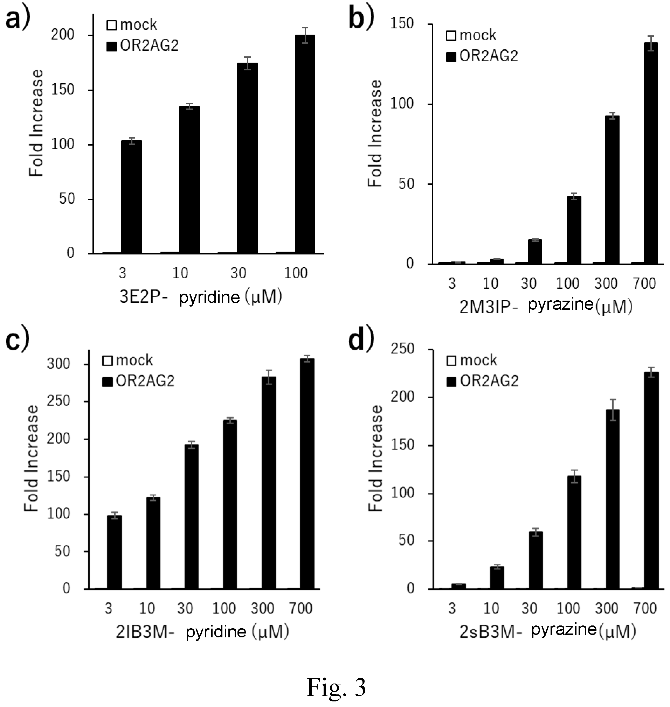

The present inventors conducted a screening of 396 human olfactory receptors for 2-isobutyl-3-methoxypyrazine, a representative substance of the "dust odor," in order to elucidate the chemical entity of an odor that is sensorially, abstractly, or subjectively expressed as "dust odor," and identified that 2-isobutyl-3-methoxypyrazine, the representative substance of the "dust odor," exhibits a strong and specific response to olfactory receptor OR2AG2 (Figure 1). Furthermore, the present inventors screened the responses of the olfactory receptor OR2AG2 to as many as 1,367 odorant substances having various structures and odor notes, and summarized the relationship between OR2AG2 responses and odor notes. As a result, the following novel findings were obtained: (i) many odorant substances that respond to OR2AG2 exhibit characteristics of the "dust odor," (ii) the odorant substances that respond particularly strongly are all compounds with strong "dust odor" characteristics, and especially (iii) a group of compounds represented by the following formula (1) or (2) exhibits strong responses to OR2AG2 (Figures 2 and 3): 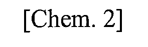 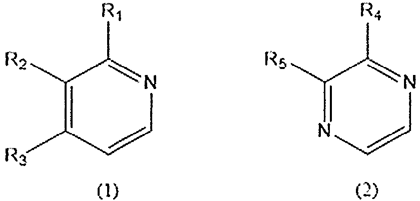
wherein in formula (1), R₁ represents a hydrogen atom, an unbranched alkyl group having 1 to 3 carbon atoms, an optionally branched alkyl group having 4 to 6 carbon atoms, or an alkoxy group having 1 to 3 carbon atoms; R₂ and R₃ each independently represent a hydrogen atom, an optionally branched alkyl group having 1 to 6 carbon atoms, or an alkoxy group having 1 to 3 carbon atoms; and
in formula (2), R₄ and R₅ each independently represent a hydrogen atom, an optionally branched alkyl group having 1 to 6 carbon atoms, or an alkoxy group having 1 to 3 carbon atoms.

Here, examples of the optionally branched alkyl group having 1 to 6 carbon atoms include methyl, ethyl, propyl, isopropyl, n-butyl, isobutyl, sec-butyl, tert-butyl, n-pentyl, isopentyl, sec-pentyl, tert-pentyl, neopentyl, n-hexyl, 1-methylpentyl, 2-methylpentyl, 3-methylpentyl, and 2-ethylbutyl. Of these, methyl, ethyl, isopropyl, isobutyl, sec-butyl, and n-pentyl are preferred.

Examples of the unbranched alkyl group having 1 to 3 carbon atoms include methyl, ethyl, and propyl.

Examples of the optionally branched alkyl group having 4 to 6 carbon atoms include n-butyl, isobutyl, sec-butyl, tert-butyl, n-pentyl, isopentyl, sec-pentyl, tert-pentyl, neopentyl, n-hexyl, 1-methylpentyl, 2-methylpentyl, 3-methylpentyl, and 2-ethylbutyl.

Examples of the alkoxy group having 1 to 3 carbon atoms include methoxy, ethoxy, propoxy, and isopropoxy.

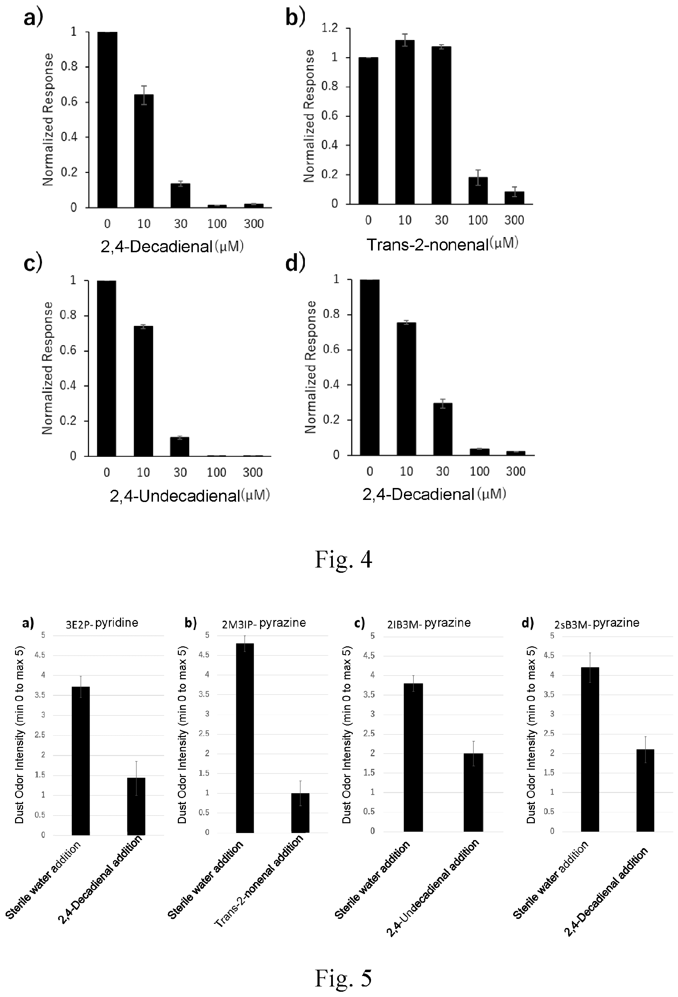

In addition, the present inventors have found that antagonists that block the response of the olfactory receptor OR2AG2 have the effect of suppressing "dust odor" (Figures 4 and 5), indicating that the olfactory receptor OR2AG2 is a narrowly-tuned olfactory receptor that is strongly associated with the recognition of "dust odor."

Based on these findings, it has been identified that the chemical entity of the odor that has hitherto been expressed sensorially, abstractly, or subjectively as "dust odor" is an odor that triggers olfactory receptor OR2AG2 response.

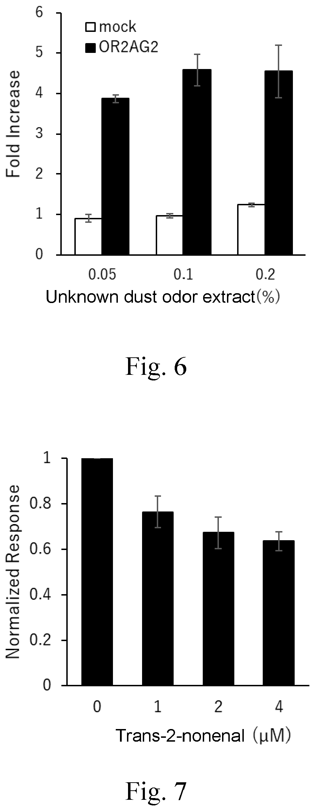

The present inventors further conducted studies and have found that, by confirming the response of the olfactory receptor OR2AG2, searching for antagonists of the olfactory receptor OR2AG2, and utilizing their masking effect, it is possible to evaluate and select odor-suppressing materials, not only for individual "dust odor"-causing substances but also for "dust odor" with unknown components (Figures 6, 7, and 8).

The present invention has been made based on these novel findings.

Hereinafter, the method for screening candidate substances for an odor-suppressing material according to the present invention (hereinafter referred to as the screening method of the present invention) will be described in detail.

As described above, the screening method of the present invention is a method for screening odor-suppressing materials, the method comprising:
causing a test substance and an odor to act on at least one olfactory receptor polypeptide selected from the group consisting of olfactory receptor OR2AG2 and polypeptides that comprise an amino acid sequence having 80% or more identity with the amino acid sequence of the olfactory receptor and are responsive to odor-causing substances;
measuring the response of the olfactory receptor polypeptide to the odor; and
on the basis of the measured response, identifying a test substance that suppresses the response of the olfactory receptor polypeptide as a candidate substance for an odor-suppressing material.

In the present invention, the term "odor" refers to an odor for which a candidate substance for a suppressing material can be screened by the method described above, and more preferably refers to an odor expressed as "dust odor."

The present invention can be carried out, for example, in accordance with the following embodiments.

As one embodiment, the present invention is characterized by being a method for screening candidate substances for a "dust odor"-suppressing material from among test substances using an olfactory receptor that is responsive to "dust odor"-causing substances, the method comprising:
(i) a step of causing a "dust odor" to act on an olfactory receptor selected from the group consisting of olfactory receptor OR2AG2 and proteins (polypeptides) that comprise an amino acid sequence having 80% or more identity with the amino acid sequence of the olfactory receptor and that are responsive to "dust odor"-causing substances, and measuring the baseline response of the olfactory receptor prior to exposure to the "dust odor"-causing substance (T0) and the response of the olfactory receptor to the "dust odor" at an arbitrary time after exposure to the "dust odor"-causing substance (Tx);
(ii) a step of calculating and determining the change value in responsiveness by comparing the baseline response of the olfactory receptor prior to exposure to the "dust odor"-causing substance (T0) with the response of the olfactory receptor to the "dust odor" at an arbitrary time after exposure to the "dust odor"-causing substance (Tx), as measured in step (i);
(iii) a step of causing a test substance to act on the "dust odor"-causing substance of step (i) and calculating and determine the change value in responsiveness in the same manner as in step (ii); and
(iv) a step of selecting a test substance for which the value of step (iii) is reduced compared to step (ii) as a candidate substance for a "dust odor"-suppressing material.

The screening method of the present invention selects a candidate substance for an odor- or "dust odor"-suppressing material from among test substances, using, as an index, the responsiveness of the test substances to an olfactory receptor selected from the group consisting of olfactory receptor OR2AG2 and proteins (polypeptides) that comprise an amino acid sequence having 80% or more identity with the amino acid sequence of the olfactory receptor and that are responsive to odor- or "dust odor"-causing substances.

It is generally known that the activation of a plurality of olfactory receptors by a single odorant substance induces emotions and behaviors such as likes and dislikes, attraction and aversion, and Non Patent Literature 7 has shown that each individual olfactory receptor defines the "quality" of an odor. By measuring the response of OR2AG2 to as many as 1,367 odorant substances having various structures and various odor notes, and by extensively studying and compiling the relationship between the OR2AG2 response and odor notes, it was found that substances that respond to OR2AG2 possess, to no small extent, odor elements of "dust odor," and that antagonist compounds of OR2AG2 reduce the unpleasantness of "dust odor," indicating that OR2AG2 is a narrowly-tuned olfactory receptor associated with the "quality" of unpleasant odors ("dust odor") that evoke emotions such as dislike or aversion. Therefore, by evaluating the responsiveness of test substances to olfactory receptor OR2AG2, it is possible to select a candidate substance from the test substances that can prevent the "dust odor" from acting on the olfactory receptor OR2AG2.

In the present specification, the term "test substance" is not particularly limited, and refers to a subject for investigation of odor-suppressing effects, and refers to a compound, composition, or mixture. In the present specification, the term "odor-suppressing material" is not particularly limited, and refers to a compound, composition, or mixture capable of suppressing an odor. Hereinafter, each step (steps (i) to (iv) described above) of the screening method of the present invention will be described.

### <Step (i)>

In step (i), a "dust odor"-causing substance is caused to act on an olfactory receptor selected from the group consisting of olfactory receptor OR2AG2 and proteins (polypeptides) that comprise an amino acid sequence having 80% or more identity with the amino acid sequence of the olfactory receptor and that are responsive to "dust odor"-causing substances, and the baseline response of the olfactory receptor prior to exposure to the "dust odor"-causing substance (T0) and the response of the olfactory receptor to the "dust odor" at an arbitrary time after exposure to the "dust odor"-causing substance (Tx) are measured. In order to appropriately compare the measurement results at the baseline response (T0) and the measurement results at the response (Tx), it is preferable that, except for the presence or absence of exposure to the "dust odor"-causing substance, the measurement conditions be the same.

The olfactory receptor used may be an olfactory receptor selected from the group consisting of OR2AG2 and proteins (polypeptides) that comprise an amino acid sequence having 80% or more identity with the amino acid sequence of OR2AG2 and that are responsive to "dust odor"-causing substances.

OR2AG2 is a protein registered in GenBank as NM_001004490, consisting of the amino acid sequence (SEQ ID No: 2) encoded by the DNA at positions 893 to 1843 of the base sequence shown in SEQ ID NO: 1.

Since the olfactory receptor OR2AG2 selectively responds to "dust odor"-causing substances, a screening method using OR2AG2 is expected to contribute to the development of "dust odor"-suppressing materials.

The olfactory receptor used may be an olfactory receptor selected from the group consisting of proteins that comprise an amino acid sequence having 80% or more, preferably 85% or more, more preferably 90% or more, still more preferably 95% or more, and particularly preferably 98% or more identity with the amino acid sequence of OR2AG2, and that are responsive to "dust odor"-causing substances. In the present specification, sequence identity of the amino acid sequence is calculated using the BLAST search algorithm (publicly available from NCBI). In addition, "being responsive to a 'dust odor'-causing substance" means that there is a change in the response value when comparing the baseline response of the olfactory receptor prior to exposure to the "dust odor"-causing substance (T0) with the response of the olfactory receptor to the "dust odor" at an arbitrary time after exposure to the "dust odor"-causing substance (Tx).

The olfactory receptor may be used singly, or two or more olfactory receptors may be used in combination.

In the present invention, "dust odor" is a dusty odor having, optionally, at least one odor characteristic selected from the group consisting of "earthy," "grassy," "powdery," "medicinal," and "moldy," which comprises, as a primary note, at least one expression selected from the group consisting of "dusty," "earthy," and "grassy (bell pepper-like or vegetable-like)," or expressions similar thereto, as a descriptor expressing the odor characteristic, and optionally, causes discomfort to humans. Here, "expressions similar thereto" may be any expression that a person skilled in the art can recognize as a descriptor expressing the same odor characteristic, and is not particularly limited. Examples of expressions similar to "earthy" include "galbanum-like" and "smoky." In addition, examples of expressions similar to "grassy (bell pepper-like or vegetable-like)" include "vegetable-like" and "bell pepper-like". According to the present definition, for example, geosmin and 2-methylisoborneol, whose primary note is represented by the descriptor "musty/moldy," would not be recognized as "dust odor."

Examples of odor-causing substances, particularly "dust odor"-causing substances, include compounds represented by the following formula (1) or (2): 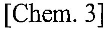 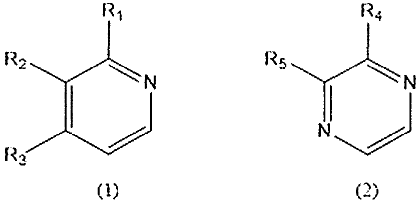
wherein in formula (1), R₁ represents a hydrogen atom, an unbranched alkyl group having 1 to 3 carbon atoms, an optionally branched alkyl group having 4 to 6 carbon atoms, or an alkoxy group having 1 to 3 carbon atoms; R₂ and R₃ each independently represent a hydrogen atom, an optionally branched alkyl group having 1 to 6 carbon atoms, or an alkoxy group having 1 to 3 carbon atoms; and
in formula (2), R₄ and R₅ each independently represent a hydrogen atom, an optionally branched alkyl group having 1 to 6 carbon atoms, or an alkoxy group having 1 to 3 carbon atoms, and can include one or more of these pyridines and pyrazines. The specific examples of the functional groups in the formulae are as described above.

In the present invention, the "dust odor" may be a "dust odor" consisting of a single "dust odor"-causing substance, or a complex odor having a "dust odor," such as an extract of a fraction having a "dust odor." As long as there is a "dust odor," a substance causing the "dust odor" is necessarily present, and it is not necessary for the substance causing the "dust odor" to be identified.

In the present invention, "causing a 'dust odor'-causing substance to act on an olfactory receptor" includes not only contacting or adding a solution of a single "dust odor"-causing substance, or a solution of a complex odor having a "dust odor," such as an extract of a fraction having a "dust odor," to olfactory receptor-expressing cells, but also exposure via the gas phase. Methods for obtaining responses by exposing olfactory receptor-expressing cells to odors are known from Non Patent Literature 8 and the like.

In the present invention, the method for causing a "dust odor"-causing substance to act on an olfactory receptor and measuring the response of the olfactory receptor to the "dust odor"-causing substance is not particularly limited. For example, the "dust odor"-causing substance may be caused to act on cells isolated from a living organism that expresses the olfactory receptor, and then the response of the olfactory receptor may be measured, or the "dust odor"-causing substance may be caused to act on the olfactory receptor on cells genetically engineered to artificially express the olfactory receptor, and then the response of the olfactory receptor may be measured. The duration for causing the "dust odor"-causing substance to act on the olfactory receptor cannot be generally specified because it depends on the concentration of the "dust odor"-causing substance. However, in the luciferase assay of the present invention, it is 0 to 4 hours, and in the calcium imaging method, it is about several seconds to several minutes.

Cells genetically engineered to artificially express an olfactory receptor can be prepared by transforming the cells using a vector incorporating a gene encoding the olfactory receptor.

In a preferred embodiment of the present invention, N-terminal 20 amino acid residues of bovine rhodopsin may be incorporated together with the olfactory receptor. Incorporation of the N-terminal 20 amino acid residues of bovine rhodopsin can promote cell membrane expression of the olfactory receptor.

Bovine rhodopsin is registered on the NCBI (GenBank) website under Accession Number NM_001014890.2 and Accession Number NP_001014890.1. Bovine rhodopsin is a protein (polypeptide) consisting of the amino acid sequence (SEQ ID No: 4) encoded by the DNA at positions 1 to 1047 of the base sequence shown in SEQ ID No: 3.

Alternatively, a protein (polypeptide) comprising an amino acid sequence having 80% or more, preferably 85% or more, more preferably 90% or more, still more preferably 95% or more, and particularly preferably 98% or more identity with the amino acid sequence shown in SEQ ID No: 4, and capable of promoting cell membrane expression of the olfactory receptor, may be used in place of bovine rhodopsin.

It should be noted that amino acid residues of other proteins (polypeptides), not limited to bovine rhodopsin, may also be used as long as they can promote cell membrane expression of the olfactory receptor.

The method for measuring the response of the olfactory receptor is not particularly limited, and any method used in the art can be used. For example, it is known that when an odorant compound binds to an olfactory receptor, it activates an intracellular G protein, which in turn activates adenylate cyclase, thereby converting ATP into cyclic AMP (cAMP) and increasing the intracellular amount of cAMP. Therefore, the response of the olfactory receptor can be measured by measuring the amount of cAMP. Methods for measuring the amount of cAMP include ELISA and reporter assay methods. Of these, it is preferable to measure the response of the olfactory receptor by a reporter assay method using a luminescent substance such as luciferase.

### <Step (ii)>

In step (ii), the change value in responsiveness is calculated and determined by comparing the baseline response of the olfactory receptor prior to exposure to the "dust odor"-causing substance (T0) with the response of the olfactory receptor to the "dust odor" at an arbitrary time after exposure to the "dust odor"-causing substance (Tx), as measured in step (i).

According to one embodiment of the present invention, the change in responsiveness may be evaluated using, as an index, the Fold Increase value determined by dividing the measurement result of the response (Tx) of the olfactory receptor to the "dust odor"-causing substance at an arbitrary time after exposure to the "dust odor"-causing substance in step (i) by the measurement result of the baseline response (T0) of the olfactory receptor prior to exposure to the "dust odor"-causing substance. For example, when measuring the response of the olfactory receptor by a reporter assay using a luminescent substance such as luciferase, the evaluation can be performed using a "dust odor" for which the Fold Increase value is preferably 2 or more, more preferably 4 or more, still more preferably 5 or more, or 10 or more, 15 or more, 20 or more, 25 or more, 30 or more, 40 or more, 45 or more, or 50 or more.

### <Step (iii)>

In step (iii), a test substance is caused to act on the "dust odor"-causing substance of step (i) and the change value in responsiveness is calculated and determined in the same manner as in step (ii). In order to appropriately compare the measurement results at the baseline response (T0) and the measurement results at the response (Tx), it is preferable that, except for the presence or absence of exposure to the "dust odor"-causing substance and test substance, the measurement conditions be the same.

### <Step (iv)>

In step (iv), a test substance for which the value of step (iii) is reduced compared to step (ii) is selected as a candidate substance for a "dust odor"-suppressing material.

In the present invention, if a reduction in the change value in responsiveness is observed by comparing the measurement results of the change values in steps (ii) and (iii), the test substance used in step (iii) can be evaluated as a candidate substance for a "dust odor"-suppressing material.

In this manner, a candidate substance for a material suppressing an odor, particularly an odor expressed as "dust odor," can be screened from among the test substances. According to the present invention, a candidate substance for a "dust odor"-suppressing material can be selected from among a large number of test substances without giving rise to problems such as olfactory fatigue or individual differences resulting from sensory evaluations performed by human olfaction.

The selected substance can be used as a candidate substance for a "dust odor"-suppressing material. Based on the selected substance, modifications and the like can also be made as necessary to develop novel compounds with optimized odors. Furthermore, it is also possible to blend the selected substance with other flavoring materials to suppress the "dust odor," and to develop flavoring materials with optimized odors. By using the screening method of the present invention, it is possible to contribute to the development of novel flavoring materials as "dust odor"-suppressing materials.

### Examples

Hereinafter, the present invention will be described in further detail with reference to Examples, but the present invention is not limited in any way to these Examples.

### [Example 1]

### Identification of olfactory receptor that responds to representative "dust odor" substance: 2-isobutyl-3-methoxypyrazine

### (1-1) Cloning of olfactory receptor genes

Human olfactory receptor genes were obtained by cloning via PCR from Human Genomic DNA: Female (Promega) based on the base sequence information registered on the aforementioned NCBI (GenBank) website (see SEQ ID No: 1). A human olfactory receptor gene expression vector was obtained by inserting into a pME18S vector DNA encoding the N-terminal 20 amino acid residues of bovine rhodopsin (DNA consisting of bases 1 to 60 of the base sequence of SEQ ID No: 3), and further inserting the obtained human olfactory receptor gene downstream thereof.

### (1-2) Expression of olfactory receptor gene in HEK293T cells

0.05 µg of the human olfactory receptor gene expression vector, 0.01 µg of RTP1S vector, 0.01 µg of the firefly luciferase vector pGL4.29 (Promega) containing a cAMP response element promoter, and 0.005 µg of the Renilla luciferase vector pGL4.74 (Promega) containing a thymidine kinase promoter were dissolved in 10 µL of Opti-MEM I (gibco) to prepare a gene solution (for one well). HEK293T cells were seeded at 100 µL per well in a 96-well plate (Biocoat, Corning) at a cell number that would reach confluence after 24 hours. The gene solution was added to each well using lipofection in accordance with the instructions for Lipofectamine 3000 to introduce the gene into the cells, which were then cultured at 37°C in a 5% carbon dioxide atmosphere for 24 hours.

### (1-3) Luciferase reporter gene assay

After removing the culture medium, 60 µL of an odorant compound sample prepared in CD293 medium (gibco) (supplemented with 20 µM L-glutamine), at the concentration required for measurement, was added to each well. After stimulation for 3 hours, luciferase activity was measured in accordance with the instructions for the Dual-Luciferase Reporter Assay System (Promega). The response intensity of the olfactory receptor was determined using, as an index, a Fold Increase value obtained by dividing the luciferase activity generated by stimulation with the odorant compound by the luciferase activity generated in a test system free of the odorant compound.

### (1-4) Olfactory receptor screening for 2-isobutyl-3-methoxypyrazine

Based on (1-1) to (1-3) above, HEK293 cells individually expressing each of 396 human olfactory receptors were prepared, to which 1 mM of 2-isobutyl-3-methoxypyrazine was added to determine the response of each of the 396 olfactory receptors as a Fold Increase value. The results are shown in Figure 1.

### [Example 2]

Demonstration that the olfactory receptor OR2AG2 is an odor-note-specific receptor for "dust odor"-causing substances

### (2-1) Screening of odorant substances that respond to OR2AG2

A human olfactory receptor OR2AG2 gene expression vector was cloned, and a total of 1,367 odorant substances were individually adjusted and added to HEK293T cells expressing the olfactory receptor OR2AG2 gene at the maximum appropriate concentration within the final concentration range of 1 µM to 1 mM that does not cause cytotoxicity, followed by a luciferase reporter gene assay. All 1,367 odorant substances used were annotated with odor-note information by odor experts. For odorant substances whose response values in the luciferase reporter gene assay were equal to or greater than a predetermined threshold, the primary note from the odor-note information was extracted, and the proportion of each odor note was determined. The results are shown in Figure 2.

### (2-2) Confirmation that "dust odor" compounds respond to OR2AG2 in concentration-dependent manner

The human olfactory receptor OR2AG2 gene expression vector was cloned, and to HEK293T cells expressing the olfactory receptor OR2AG2 gene was added representative samples of odorant substances having "dust odor," which are compounds that trigger OR2AG2 response, as shown in Figure 2: a) 3-ethyl-2-pentylpyridine, b) 2-methoxy-3-isopropylpyrazine, c) 2-isobutyl-3-methoxypyrazine, and d) 2-sec-butyl-3-methoxypyrazine, at final concentrations of 3 µM, 10 µM, 30 µM, 100 µM, 300 µM, and 700 µM, followed by a luciferase reporter gene assay. The results are shown in Figure 3.

### (2-3) Identification of OR2AG2 antagonists for "dust odor" compounds

The human olfactory receptor OR2AG2 gene expression vector was cloned, and HEK293T cells expressing the olfactory receptor OR2AG2 gene were used to perform an antagonist screening in which the effect of test substances on suppressing OR2AG2 response is measured by a luciferase reporter gene assay. In the luciferase reporter gene assay, each of approximately 500 test substances was individually mixed with each "dust odor" compound sample, and the Fold Increase value obtained in each test with the test substance mixed was calculated as the Normalized Response, assuming that the Fold Increase value obtained in the test without mixing any test substance was 1. The concentrations of the "dust odor" compounds used as samples were set as follows: a) 3-ethyl-2-pentylpyridine, 300 µM; b) 2-methoxy-3-isopropylpyrazine, 500 µM; c) 2-isobutyl-3-methoxypyrazine, 500 µM; and d) 2-sec-butyl-3-methoxypyrazine, 500 µM. The concentrations of the test substances were each appropriately adjusted within the final concentration range of 1 µM to 1 mM that does not cause cytotoxicity. Test substances whose Normalized Response is less than 1 can be considered antagonist substances that suppress OR2AG2 response, and test substances exhibiting lower Normalized Response values can be considered strong antagonist substances. As examples of antagonists thereby identified for each of the "dust odor" compounds a) to d), a) 2,4-decadienal, b) trans-2-nonenal, c) 2,4-undecadienal, and d) 2,4-decadienal were measured for their concentration-dependent effects in suppressing OR2AG2 response to each sample substance using a luciferase reporter gene assay. The concentrations of the samples were set as follows: a) 3-ethyl-2-pentylpyridine, 300 µM; b) 2-methoxy-3-isopropylpyrazine, 500 µM; c) 2-isobutyl-3-methoxypyrazine, 500 µM; and d) 2-sec-butyl-3-methoxypyrazine, 500 µM. The concentrations of the antagonists for each sample were set at 0 µM, 10 µM, 30 µM, 100 µM, and 300 µM. The results are shown in Figure 4.

### (2-4) Evaluation of "dust odor"-masking ability by OR2AG2 antagonists

The ability of OR2AG2 antagonists to suppress "dust odor" for each sample having "dust odor" was confirmed by sensory evaluation. For each sample: a) 3-ethyl-2-pentylpyridine, b) 2-methoxy-3-isopropylpyrazine, c) 2-isobutyl-3-methoxypyrazine, and d) 2-sec-butyl-3-methoxypyrazine, 10 µL of a 100 mM aqueous solution was added dropwise onto Muette paper, and simultaneously, 10 µL of sterile water (control) or 10 µL of the OR2AG2 antagonist for each sample: a) 2,4-decadienal, b) trans-2-nonenal, c) 2,4-undecadienal, and d) 2,4-decadienal at 200 µM, was added dropwise. The sensory evaluation test was conducted with a panel of five or more individuals (sample a: 7 and samples b to d: 5). The odor intensity when distilled water was added dropwise to the "dust odor" compound was rated as 3 to 5, and the "dust odor" intensity when the test substance was added dropwise was rated as 0 (no odor perceived) to 5 (very strong odor perceived). The average value was calculated from the obtained values. The results are shown in Figure 5.

### [Example 3]

### Application to searching masking agents utilizing OR2AG2 response to unknown "dust odor"

### (3-1) Confirmation of OR2AG2 response to unknown "dust odor"

To extract the "dust odor" generated when aspartame is combined with certain fruit flavors, as described in Patent Literature 1, which is well-known as a "dust odor" of unknown causative substance, aspartame, trans-2-hexenal, and hexanal were mixed at a 1:1:1 (mass ratio) and gently heated at 60°C for one week, after which the reaction product was extracted with hexane. The present extract was fractionated by medium-pressure HPLC with a silica gel column using hexane and ethyl acetate, then fractionated by medium-pressure HPLC with an ODS column using water and acetonitrile, and further fractionated by high-pressure HPLC with an ODS column using water and acetonitrile, to obtain an odor solution containing "dust odor." This "dust odor" solution with unknown components was added to HEK293T cells expressing the olfactory receptor OR2AG2 gene at final concentrations of 0.05 mass%, 0.1 mass%, and 0.2 mass%, and a luciferase reporter gene assay was performed. The results are shown in Figure 6.

### (3-2) Identification of OR2AG2 antagonists for unknown "dust odor"

As in (2-3) of Example 2, HEK293T cells expressing the olfactory receptor OR2AG2 gene were used to determine the effect of test substances in suppressing OR2AG2 response to an odor solution sample with unknown components containing "dust odor" by antagonist screening using a luciferase reporter gene assay. To a 0.1 mass% odor solution sample with unknown components containing "dust odor" were mixed approximately 500 test substances at final concentrations of 1 µM to 1 mM, and the Normalized Response was calculated to obtain OR2AG2 antagonists for the "dust odor" with unknown components that had a Normalized Response below 1. As an example of antagonist, the effect of trans-2-nonenal in suppressing OR2AG2 response to the "dust odor" solution with unknown components was measured by a luciferase reporter gene assay. The final concentration of the sample was 0.1 mass%, and the concentrations of trans-2-nonenal relative to the sample were 0 µM, 1 µM, 2 µM, and 4 µM. The results are shown in Figure 7.

### (3-3) Evaluation of masking ability of OR2AG2 antagonists against "dust odor" with unknown components

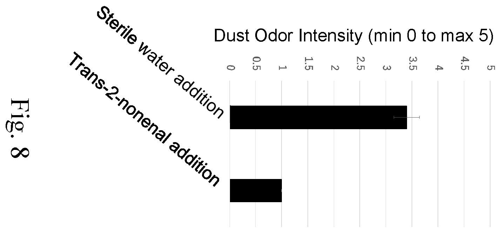

Ten microliters of the "dust odor" solution with unknown components were added dropwise onto Muette paper, and simultaneously, 10 µL of sterile water (control) or 10 µL of 200 µM trans-2-nonenal was added dropwise. The sensory evaluation test was conducted with a panel of five individuals. The odor intensity when distilled water was added dropwise to the "dust odor" solution was rated as 3 to 5, and the "dust odor" intensity when trans-2-nonenal was added dropwise was rated as 0 (no odor perceived) to 5 (very strong odor perceived). The average value was calculated from the obtained values. The results are shown in Figure 8.

### Results of the Examples

Responses to 2-isobutyl-3-methoxypyrazine (1 mM) as a representative substance of "dust odor" were measured for 396 olfactory receptors, and as a result, olfactory receptor OR2AG2 exhibited an extremely strong response (Figure 1). The responses of OR2AG2 to as many as 1,367 odorant substances with various structures and odor notes were specific to "dust odor" (Figure 2). A group of compounds represented by formula (1a) or (1b) in particular elicited responses from OR2AG2, and the OR2AG2 responses to various "dust odor" compounds were concentration-dependent (Figure 3). The OR2AG2 responses to various "dust odor" compounds were suppressed in a concentration-dependent manner by OR2AG2 antagonists (Figure 4), and in human living organisms, the OR2AG2 antagonists markedly reduced the perception of "dust odor" from the various "dust odor" compounds (Figure 5). These results indicate that "dust odor" is recognized via OR2AG2. OR2AG2 is a novel "dust odor" receptor whose physiological function has not previously been identified.

Similarly, even a mixed extract containing "dust odor" with unknown components elicited a concentration-dependent response in OR2AG2, as long as it possessed a "dust odor" (Figure 6). The OR2AG2 response to "dust odor" with unknown components was suppressed in a concentration-dependent manner by an OR2AG2 antagonist (Figure 7), and the perception of "dust odor" was markedly reduced (Figure 8). This indicates that even when the causative substance is unidentified, candidate substances for "dust odor"-suppressing materials can be screened using the OR2AG2 response.

### Industrial Applicability

By using the screening method of the present invention, a candidate substance for an odor-suppressing material, particularly a "dust odor"-suppressing material, can be selected from among a large number of test substances. The screening method of the present invention is expected to contribute to the development of odor-suppressing materials, particularly "dust odor"-suppressing materials.

## Claims

1. A method for screening odor-suppressing materials, the method comprising:
causing a test substance and an odor-causing substance to act on at least one olfactory receptor polypeptide selected from the group consisting of olfactory receptor OR2AG2 and polypeptides that comprise an amino acid sequence having 80% or more identity with the amino acid sequence of the olfactory receptor and that are responsive to odor-causing substances;
measuring the response of the olfactory receptor polypeptide to the odor-causing substance; and
on the basis of the measured response, identifying a test substance that suppresses the response of the olfactory receptor polypeptide as a candidate substance for an odor-suppressing material.

2. The method according to claim 1, wherein the odor is an odor expressed as "dust odor,"
has at least one odor characteristic selected from the group consisting of dusty, and optionally, earthy, grassy, powdery, medicinal, and moldy,
is an odor that comprises, as a primary note, at least one expression selected from the group consisting of "dusty," "earthy," and "grassy (bell pepper-like or vegetable-like)," or expressions similar thereto, as a descriptor expressing the odor characteristic, and
optionally, is an odor that causes discomfort to humans.

3. The method according to claim 1, wherein the odor is an odor that triggers olfactory receptor OR2AG2 response.

4. The method according to claim 1, wherein the odor-causing substance comprises one or more selected from the group consisting of compounds represented by the following formula (1) or (2): 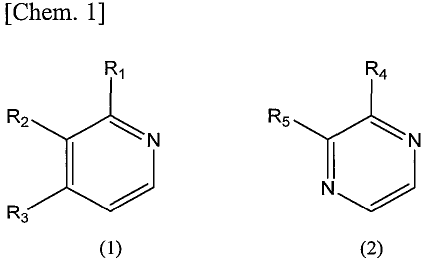
wherein in formula (1), R₁ represents a hydrogen atom, an unbranched alkyl group having 1 to 3 carbon atoms, an optionally branched alkyl group having 4 to 6 carbon atoms, or an alkoxy group having 1 to 3 carbon atoms; R₂ and R₃ each independently represent a hydrogen atom, an optionally branched alkyl group having 1 to 6 carbon atoms, or an alkoxy group having 1 to 3 carbon atoms; and
in formula (2), R₄ and Rs each independently represent a hydrogen atom, an optionally branched alkyl group having 1 to 6 carbon atoms, or an alkoxy group having 1 to 3 carbon atoms.

5. The method according to claim 1, wherein the response of the olfactory receptor polypeptide to the odor-causing substance is measured on cells that endogenously express the olfactory receptor, or on cells genetically engineered to artificially express the olfactory receptor.

6. The method according to claim 1, wherein the response of the olfactory receptor polypeptide is measured by a reporter assay or calcium imaging.
